# EUROPÄISCHE PATENTSCHRIFT

(11) **EP 0 388 745 B1**
(45) Veröffentlichungstag und Bekanntmachung des Hinweises auf die Patenterteilung: **10.02.1993**
(21) Anmeldenummer: 90104602.9
(22) Anmeldetag: 12.03.1990
(51) Int. Cl.: A61F 2/34

(54) **Hüftgelenkspfanne zur zementfreien Implantation in das Acetabulum des Hüftbeines**
Hip joint cup for cementless implantation into the acetabulum cavity
Cuvette d'articulation de la hanche pour l'implantation sans ciment dans la cavité acétabulaire

(30) Priorität: 17.03.1989 DE 8903328 U
(43) Veröffentlichungstag der Anmeldung: 26.09.1990
(73) Patentinhaber: Thull, Roger, Prof. Dr.-Ing., D-97082 Würzburg (DE)
(72) Erfinder: Thull, Roger, Prof.-Dr.-Ing., D-8700 Würzburg (DE); Zeiler, Günther, Dr.- med., D-8503 Altdorf (DE)
(74) Vertreter: Hufnagel, Walter, Dipl.-Ing., Dipl.-Wirtsch.-Ing.

(56) Entgegenhaltungen:
- EP-A- 0 169 978
- EP-A- 0 187 881
- EP-A- 0 225 819

## Beschreibung

Die Erfindung betrifft eine Hüftgelenkspfanne zur zementfreien Implantation in das Acetabulum des Hüftbeins, bestehend aus einem elastischen Pfannenträger, der die Form einer im wesentlichen halbkugelförmigen Schale aufweist und in das entsprechend vorbereitete Acetabulumbett formschlüssig einsetzbar ist gemäß dem Oberbegriff des Anspruches 1.

Eine Hüftgelenkspfanne ist der in das Hüftbein eines Patienten zu implantierende Teil eines Hüftgelenksprothesensystems, während der andere, aus einem Schaft und einem, einen Kugelkopf tragenden Halsstück bestehende Teil, den in den Oberschenkelknochen zu implantierenden Teil dieses Systems darstellt.

Eine solche Hüftgelenkspfanne enthält häufig einen aus Metall gefertigten Pfannenträger, der in das entsprechend vorbereitete Acetabulumbett einsetzbar und dort befestigbar ist, sowie eine in die Konkavseite des Pfannenträgers formschlüssig einsetzbare Innenpfanne aus Kunststoff, deren Konkavseite als Lager für den Kugelkopf der Hüftgelenksprothese dient.

Aus einem Firmenprospekt der Firma PROTEK AG, CH-3001 Bern, Ausgabe 1988/1 bzw. aus der gattungsbildenden EP-B 0 169 978 ist ein zementfreies Hüfttotalprothesensystem CLS bekannt, bei dem der Pfannenträger aus einer rotationssymmetrischen Halbkugelschale besteht, die sechs radiale Einschnitte aufweist, die den größten Teil der Halbkugelschale durchsetzen. Auf diese Weise entstehen sechs sternförmig angeordnete, zum Äquator hin zunehmend breiter werdende Lappen. Auf diesen Lappen befinden sich auf drei Reihen radial angeordnete, nach außen gerichtete Verankerungsspitzen.

Zum Implantieren dieses Pfannenträgers in das vorbereitete Acetabulumbett werden die elastischen Lappen mit einer Spannzange zusammengepreßt und der Pfannenträger im Acetabulum positioniert. Beim Lösen der Spannzange werden durch die radial nach außen wirkende Spannung die äquatornahen Bereiche des Pfannenträgers an den Knochen angepreßt. Mit einem Spreizkonus oder mit der Kunststoffinnenpfanne wird anschließend der Pfannenträger aufgespreizt, so daß die Verankerungsspitzen in den Knochen eingedrückt werden und damit eine stabile Primärverankerung erreicht wird.

Eine Sekundärverankerung wird durch das Anwachsen von Knochensubstanz an Vorsprüngen der äußeren Oberfläche des Pfannenträgers ermöglicht, die als fest verankerte hinterschneidungsfreie Zotten ausgebildet sind.

Die bei dem bekannten Pfannenträger vorhandene Elastizität dient ausschließlich dazu, diesen mit Hilfe der Spannzange in das Acetabulum einsetzen zu können. Um diese Elastizität zu erreichen, ist die Wandstärke im Übergangsbereich zwischen den Verankerungspitzen und dem Pol sehr dünn. Da andererseits der Pfannenträger im implantierten Zustand die Symmetrieachse des Pfannenträgers in einem Winkel von etwa 45° zur Körperachse des Patienten geneigt ist, sind die beim stehenden Patienten von dem Kugelkopf der Gelenksprothese auf den Pfannenträger übertragenen Kräfte gerade dort am größten, wo der Pfannenträger seine schwächsten Stellen hat. Dadurch besteht die Gefahr, daß der Pfannenträger sich verformt, so daß bereits bei normaler Belastung, wie sie beispielsweise beim Gehen auftritt, sowohl die Primär- als auch die Sekundärverankerung sich lockern kann. In jedem Fall wird eine feste Verbindung zum Knochen behindert.

Ein weiterer Nachteil besteht darin, daß der bekannte Pfannenträger nur schwierig und teuer herstellbar ist, insbesondere wegen der zahlreichen Verankerungsspitzen, die einzeln bearbeitet werden müssen.

Der Erfindung liegt demgegenüber die Aufgabe zugrunde, eine auf billige Weise durch einfache Dreh- und Fräsvorgänge zu bearbeitende Hüftgelenkspfanne der eingangs erwähnten Art zu schaffen, die nach ihrer Implantation schneller mit dem Hüftbeinknochen verwächst und insbesondere auch bei größeren Belastungen eine Relativbewegung zwischen dem Pfannenträger und dem Acetabulum verhindert.

Diese Aufgabe wird durch die kennzeichnenden Merkmale des Anspruches 1 gelöst.

Der aus Metall bestehende Pfannenträger ist also im oberen Bereich, der der Außenfläche etwa einer Viertelkugel entspricht, massiv ausgelegt, während im unteren Bereich die verbleibende Viertelkugel weitestgehend elastisch, mit vom Ort abhängiger Flexibilität, ausgebildet ist.

Nach einer Weiterbildung der Erfindung verlaufen die parallel oder wenigstens annähernd parallel zu der Äquatorebene ausgerichteten Einschnitte bis zur oder bis in die Nähe der Symmetrieachse oder auch geringfügig darüber hinaus in verschiedenen Abständen von der Äquatorebene, wobei eine oder mehrere Spangen gebildet sind, die durch einen radialen Einschnitt in mehrere Spangenteile aufgeteilt sind. Mit dieser Ausgestaltung erhält man in dem elastischen Teil des Pfannenträgers hocheleastische Spangenteile, deren Elastizität zunächst durch die Zahl der Einschnitte und der dadurch bedingten Spangenteilbreiten steuerbar ist.

Besonders vorteilhaft ist es, wenn die Dicke der Spangenteile beiderseits des radialen Einschnitts jeweils bis zum Ende der Spangenteile zunimmt, vorzugsweise stufenlos zunimmt. Mit dieser Maßnahme kann die Elastizität der Spangenteile noch weiter den spezifischen Bedürfnissen angepaßt werden. Insbesondere kann dadurch eine gleichmäßige Biegebeanspruchung der Spangenteile über deren äquatoriale Erstreckung eingestellt werden.

Um die Herstellung der erfindungsgemäßen Hüftgelenkspfannen auf Drehautomaten zu erleichtern, ist nach einer weiteren Ausgestaltung der Erfindung vorgesehen, daß die Innenfläche und die Außenfläche des Pfannenträgers die Form von Halbkugeln mit sich deckenden Äquatorebenen, jedoch mit unterschiedlichen Durchmessern und mit gegeneinander parallelverschobenen Symmetrieachsen aufweisen. Diese Halbkugelform dient ferner dazu, um irgendwelche Kanten, wie sie bei zylindrischer oder konischer Außenform des Pfannenträgers entstehen, zu vermeiden, die die ständige Gefahr bilden würden, aufgrund der hohen, an diesen Stellen auf den Knochen wirkenden Drucke, die tragenden Knochenstrukturen zu verletzen bzw. durch Knochenumbau zu schwächen.

Vorteilhaft zum Erreichen einer der natürlichen Pfanne entsprechenden Flexibilität sind drei Einschnitte. Die zwischen diesen Einschnitten entstehenden Spangen dienen dabei gleichzeitig als Fixierelemente für den einwachsenden Knochen, so daß einerseits eine mechanisch feste, andererseits aber auch eine elastische Verankerung des Knochens in dem elastischen Pfannenträger ermöglicht wird.

Schließlich ist es nach einer weiteren Ausgestaltung der Erfindung auch von Vorteil, daß in dem Pfannenträger eine Innenpfanne aus Kunststoff, vorzugsweise aus Polyäthylen, formschlüssig einsetzbar ist, die durch einen auf den Rand des Pfannenträgers aufliegenden Kragen am Verschwenken um die in der Äquatorebene liegenden Achsen gehindert ist.

Weitere vorteilhafte Eigenschaften der vorliegenden Erfindung sind in den Unteransprüchen beansprucht und werden anhand von Ausführungsbeispielen im folgenden näher beschrieben.

Es zeigen:
Fig. 1 eine auseinandergezogene Darstellung einer Hüftgelenkspfanne in ihrer Gebrauchslage,
Fig. 2 eine Seitenansicht des Pfannenträgers gemäß dem Schnitt I-I der Fig. 4,
Fig. 3 eine Rückansicht dieses Pfannenträgers,
Fig. 4 eine Draufsicht auf diesen Pfannenträger,
Fig. 5 eine Unteransicht dieses Pfannenträgers,
Fig. 6 einen Seitenschnitt durch eine Innenpfanne und
Fig. 7 die Draufsicht auf die Innenpfanne gemäß Fig. 5 in verkleinertem Maßstab

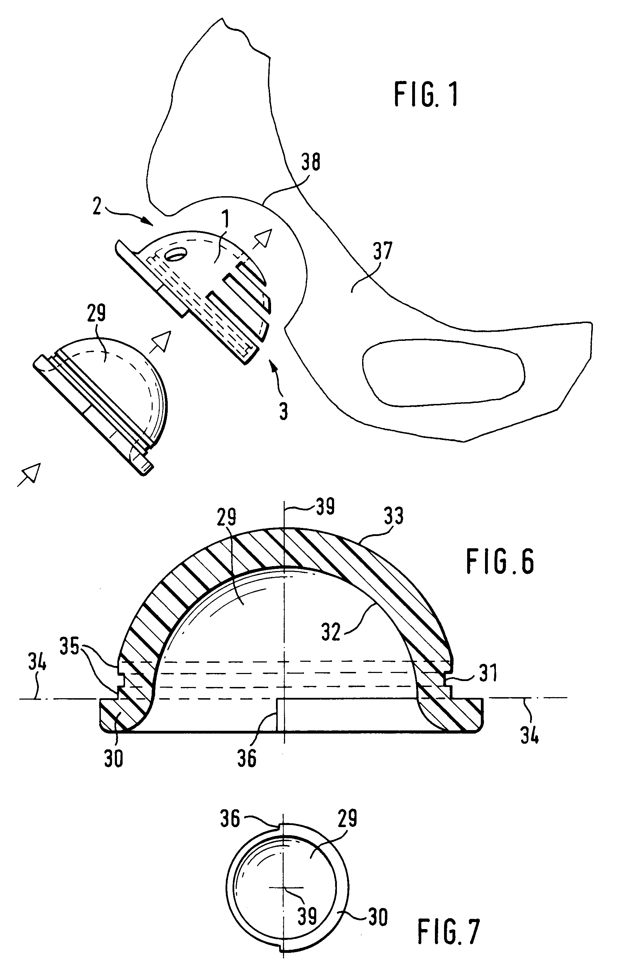

Fig. 1 zeigt ein Hüftbein 37 mit dem Acetabulum 38 - mit dem Begriff "Acetabulum" wird in dieser Anmeldung grundsätzlich die natürliche Hüftpfanne bezeichnet - ferner einen elastischen Pfannenträger 1, der in das entsprechend vorbereitete, vorzugsweise ausgefräste Acetabulumbett formschlüssig einsetzbar ist. In diesen Pfannenträger 1, der aus Metall, vorzugsweise aus Titan, besteht, kann nach seiner Implantation eine aus Kunststoff, bevorzugt aus Polyäthylen hergestellte Innenpfanne 29 eingesetzt und im Pfannenträger 1 befestigt werden.

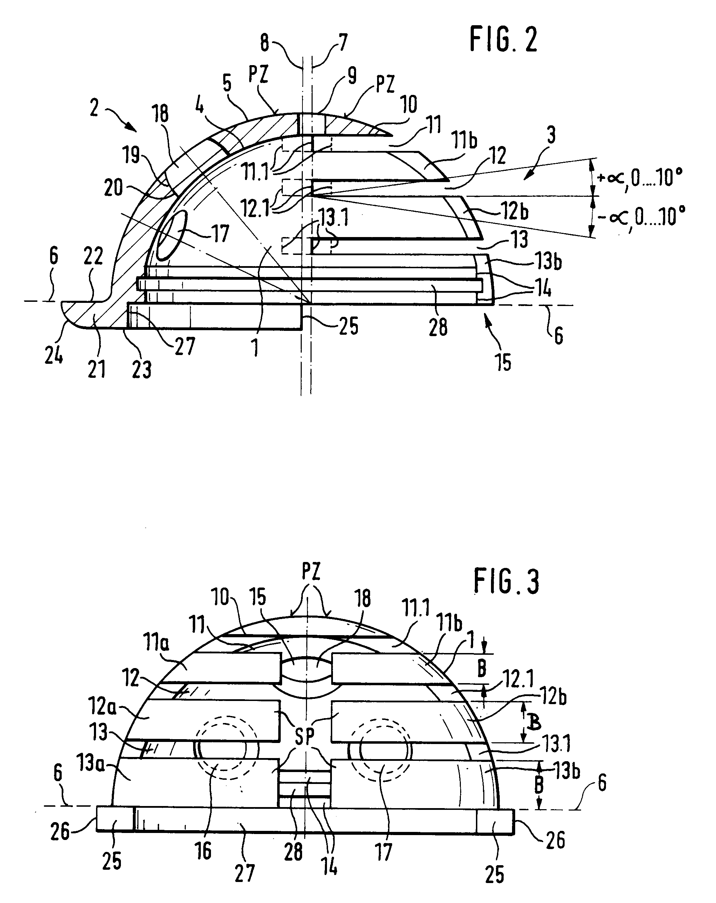

Wie aus Fig. 1 zu ersehen ist, weist der Pfannenträger 1 in dem im implantierten Zustand nach oben (kranial) gerichteten Bereich 2 seine größte Steifigkeit auf, während in Richtung zum unteren Bereich 3 (kaudal) die Elastizität des Pfannenträgers 1 zunimmt. Das wird einerseits dadurch erreicht, daß die Wandstärke des Pfannenträgers 1 im oberen Bereich 2 am größten ist, beispielsweise 3,5 mm, und in Richtung zum unteren Bereich 3 abnimmt, wobei die kleinste Wandstärke etwa 1,5 mm beträgt. Andererseits wird die Elastizität des Pfannenträgers 1 in dem Bereich mit erhöhter Elastizität noch dadurch gesteuert, daß in verschiedenen Abständen von der Äquatorebene 6 (Fig. 2) zwei oder mehr parallel oder wenigstens annähernd parallel zu der Äquatorebene 6 verlaufende Einschnitte 11, 12, 13 (Fig .2 und 3) vorgesehen sind.

In den Fig. 2 bis 5 sind weitere Einzelheiten des Pfannenträgers 1 dargestellt.

Die Innen- und die Außenfläche des Pfannenträgers 1 weisen im wesentlichen die Form zweier Halbkugeln 4, 5 mit sich deckenden Äquatorebenen 6, jedoch mit unterschiedlichen Durchmessern und mit gegeneinander parallelverschobenen Symmetrieachsen 7, 8 auf.

In dem unterhalb (in Fig. 2 rechts dargestellt) der Symmetrieachse 7 der inneren Halbkugel 4 liegenden Teil des Pfannenträgers 1 sind in verschiedenen Abständen von der Äquatorebene 6 drei parallel zu dieser verlaufende Einschnitte 11, 12, 13 vorgesehen, die sich im dargestellten Ausführungsbeispiel bis zu der durch die Symmetrieachse 7 der inneren Halbkugel 4 verlaufenden Ebene erstrecken, so daß, wie in Fig. 2 dargestellt, die inneren Begrenzungskanten 11.1, 12.1, 13.1 der Einschnitte 11, 12, 13 mit dieser Ebene zusammenfallen. Die inneren Begrenzungskanten 11.1, 12.1, 13.1 können jedoch auch hinter der genannten Ebene - wie in Fig. 2 gestrichelt dargestellt - oder vor dieser Ebene - wie in Fig. 2 durch die punktierte Linie dargestellt - verlaufen.

Vorzugsweise haben die Einschnitte 11, 12, 13 eine Breite von 1,5 mm bis 2, 5mm, wobei ihre Abstände, d.h. die Breite B der dadurch entstehenden Spangen SP, zueinander in Richtung zur Äquatorebene 6 zunehmen (Fig. 3). Die obere Schnittfläche 10 des obersten Einschnitts 11 ist so angelegt, daß sie die innere Halbkugelfläche 4 im Pfannenzentrum PZ tangential berührt. Grundsätzlich können auch mehr oder weniger als drei zueinander parallel verlaufende Einschnitte 11, 12, 13 vorgesehen sein. Auch kann es für verschiedene Anwendungsfälle vorteilhaft sein, die Einschnitte 11, 12, 13 bereits vor der Symmetrieachse 7 enden zu lassen oder auch geringfügig, d.h. mehrere Millimeter bis zu etwa 1,5 cm darüber hinaus zu erstrecken. Im ersten Fall würde die Elastizität des Pfannenträgers 1 verringert, im zweiten Fall hingegen erhöht werden.

Die Einschnitte 11, 12, 13 verlaufen bevorzugt parallel zu der Äquatorebene 6 (Fig. 2 und 3). Im Rahmen der Erfindung liegen jedoch auch Abwandlungen mit einer Neigung der Einschnitte 11, 12, 13 zu der Äquatorebene 6 bevorzugt bis zu ± 5°, maximal bis zu ± 10°. Diese Ausgestaltungen der Erfindung sind in Fig. 2 durch die eingezeichneten Winkel + α und - α angedeutet.

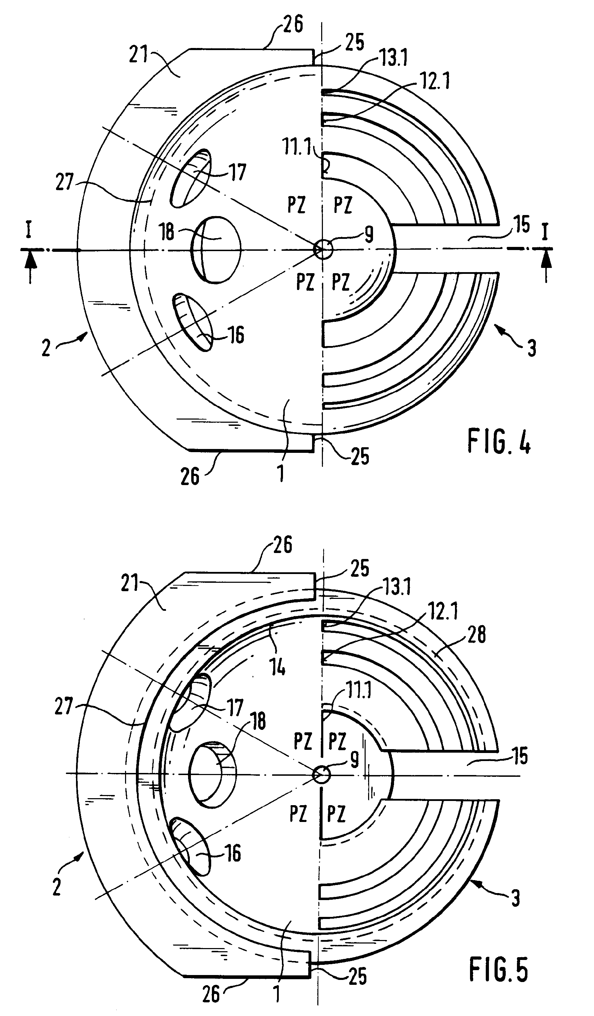

Günstig ist auch, daß der radial verlaufende Einschnitt 15 sich nur bis zu dem obersten parallel zur Äquatorebene 6 verlaufenden Einschnitt 11 erstreckt, so daß das Pfannenzentrum PZ auch im Bereich des Pfannenträgers 1 mit erhöhter Elastizität eine einschnittlose, geschlossene Fläche bildet, wie Fig. 4 zeigt.

In dem nach oben (Fig. 2 nach links) gerichteten Bereich 2 des Pfannenträgers 1 sind drei Durchbrüche 16, 17, 18 zum Aufnehmen von Befestigungsschrauben mit Senkköpfen vorgesehen, mit denen die Primärverankerung des Pfannenträgers 1 in dem Hüftbein 37 vorgenommen wird. Zwei der Durchbrüche 16, 17 sind randnah in einem Winkel von 25° und ein weiterer Durchbruch 18 ist randfern in einem Winkel von 50° über der Äquatorebene 6 angeordnet, während die Zentren der beiden randnahen Durchbrüche 16, 17 parallel zur Äquatorebene 6 um 30° nach beiden Seiten gegenüber dem randfernen Durchbruch 18 versetzt sind. Die Durchbrüche 16; 17, 18 weisen im äußeren Bereich 19 die Form einer Halbkugel und in dem nach innen anschließenden Bereich 20 die Form eines Zylinders mit 8 mm Durchmesser auf.

Die Primärverankerung des Pfannenträgers 1 im Hüftbein 37 erfolgt bevorzugt durch drei Knochenschrauben, deren Einschraubrichtung sich durch die oben beschriebene Konstruktion der Durchbrüche 16, 17, 18 auf innerhalb eines Kegels mit einem Öffnungswinkel von 10° variieren läßt. Damit lassen sich die Schrauben den anatomischen Verhältnissen und in Richtung des Kraftflusses in das Hüftbein 37 positionieren.

An dem oberhalb (in Fig. 2 links) der Symmetrieachse 8 der äußeren Halbkugel 5 liegenden Teil des Pfannenträgers 1 ist ein sich vom Rand aus nach außen erstreckender Kragen 21 vorgesehen, dessen Oberseite 22 in der Äquatorebene 6 und dessen Unterseite 23 parallel und unterhalb dieser verläuft und am äußeren Rand eine Abrundung 24 aufweist, die in einem rechten Winkel auf die Oberseite 22 trifft. Der Krümmungsradius der Abrundung 24 ist dabei gleich der Dicke des Kragens 21, nämlich vorzugsweise 3 mm, der eine Breite von etwa 6 mm hat. Der Übergang von der Oberseite 22 des Kragens 21 zur äußeren Halbkugel 5 weist eine Abrundung mit einem Krümmungsradius von etwa 1,5 mm auf.

Der Kragen 21 erstreckt sich beidseitig bis zu der durch die Symmetrieachse 8 der äußeren Halbkugel 5 verlaufenden Ebene und bildet dort radiale Endflächen 25. Ferner ist er im Bereich dieser Endflächen 25 durch zwei parallele Flächen 26 begrenzt, deren Abstand voneinander geringfügig, bevorzugt um 4 mm, größer ist als der Durchmesser des Pfannenträgers 1. Dieser Abstand kann aber auch gleich dem Durchmesser des Pfannenträgers 1 sein.

Der Kragen 21 ist in Richtung nach innen durch die Mantelfläche eines Zylinders 27 begrenzt, dessen Symmetrieachse mit der Symmetrieachse 7 der inneren Halbkugel 4 zusammenfällt und dessen Durchmesser etwa 4 mm größer ist als der Durchmesser der inneren Halbkugel 4.

Die Innenfläche des Pfannenträgers 1 geht in der Nähe der Äquatorebene 6 von der Form der inneren Halbkugel 4 in die Form eines Zylinders 14 mit einer Höhe von 4,5 mm über, dessen Symmetrieachse mit der Symmetrieachse 7 der inneren Halbkugel 4 zusammenfällt. Sie weist eine in geringem Abstand von etwa 1,5 mm von der Äquatorebene 6 umlaufende Nut 28 mit einer Tiefe von 0,8 mm und einer Breite von 1,4 mm auf, in die ein Federdraht zum Festhalten der Innenpfanne 29 einlegbar ist. Aber auch andere mechanische Verrastungen, beispielsweise nach Art von Bayonettverschlüssen oder dgl. sind möglich.

Der Pfannenträger 1 weist ferner einen zur Symmetrieachse 7 der inneren Halbkugel 4 symmetrisch angeordneten, bevorzugt zylindrischen Durchbruch 9 mit einem Durchmesser von etwa 3 mm auf, der als Zentrierhilfe bzw. zur vorläufigen Fixierung beim Einsetzen des Pfannenträgers 1 in das Hüftbein 37 dient.

Wie bereits erwähnt, besteht der Pfannenträger 1 bevorzugt aus Titan. Aber auch Pfannenträger 1 aus Titanlegierungen oder aus anderen körperverträglichen Metallen können grundsätzlich zum Einsatz kommen.

Der nach oben gerichtete Bereich 2, der sich von dem Durchbruch 9 an der Symmetrieachse 7 bis zum Kragen 21 erstreckt, sowie die Oberseite 22 des Kragens 21 sind bevorzugt mit Titanpulver oder mit Hydroxyl-Apatit beschichtet. Sie können auch, insbesondere durch hochfrequente Plasmabefestigung, mit Titan-Tantal-Oxid beschichtet sein. Vorzugsweise jedoch sind diese Flächen durch Sandstrahlen oder durch andere mechanische Methoden, wie dem PVD (Physical Vapour Deposition) Verfahren mechanisch aufgerauht und mit einer "knochenfreundlichen" Hartstoffschicht aus Oxiden, Nitriden oder Oxinitriden sogenannter "Ventilmetalle" versehen. Auch können Mischmetalloxidschichten aus Titan-Zirkon, Titan-Niob, Titan-Tantal, Titan-Haffnium, Titan-Wolfram, Titan-Tantal-Zirkon, Titan-Tantal-Niob, Titan-Tantal-Haffnium, Zirkon-Niob, Niob-Haffnium oder Tantal-Zirkon eingesetzt werden.

Der Vorteil der Oberflächenbeschichtung des an seiner Oberfläche aufgerauhten Pfannenträgers 1 liegt insbesondere darin, daß eine Rauhigkeit geschaffen wird, die eine Relativbewegung zwischen dem Pfannenträger 1 und dem anwachsenden Knochen weitestgehend verhindert. Im Gegensatz zu Pulverbeschichtungen besteht nicht die Gefahr des nachträglichen Abriebes aufgrund dieser Relativbewegungen. Vielmehr bleibt bei der nachträglichen Beschichtung der aufgerauhten Oberfläche des Pfannenträgers mit Hartmetall die Struktur der Beschichtung grundsätzlich erhalten.

Der Pfannenträger 1 wird vorzugsweise mit Durchmessern von 44 mm, 46 mm, 48 mm, 50 mm, 52 mm, 54 mm und 56 mm durch zerspanende Arbeitsgänge aus Vollmaterial hergestellt.

Fig. 6 zeigt in einem Seitenschnitt die aus Kunststoff, vorzugsweise Polyäthylen, gefertigte Innenpfanne 29, die in den Pfannenträger 1 formschlüssig einsetzbar ist und deren Innen- und Außenfläche die Form von konzentrischen Halbkugeln 32, 33 mit unterschiedlichen Durchmessern aufweisen, wobei die Außenfläche der Innenpfanne 29 in der Nähe der Äquatorebene 34 von der Form der äußeren Halbkugel 33 in die Form eines Zylinders 35 übergeht, der mit der entsprechenden Innenfläche des Pfannenträgers 1 korrespondiert.

An der Äquatorebene 34 der Halbkugel 32, 33 schließt sich ein Kragen 30 an, der auf dem Rand des Pfannenträgers 1 aufliegt, wenn die Innenpfanne 29 in den Pfannenträger 1 eingesetzt ist. Dieser Kragen 30 verhindert ein Verschwenken der Innenpfanne 29 um die in der Äquatorebene 34 liegenden Achsen.

Der Kragen 30 der Innenpfanne 29 weist etwa in der Mitte eine Stufe 36 auf, die an den radialen Endflächen 25 des Kragens 21 des Pfannenträgers 1 anliegen und auf diese Weise ein Verdrehen der Innenpfanne 29 um ihre Symmetrieachse 39 verhindern.

Die Innenfläche und die Außenfläche der Innenpfanne 29 haben die Form von konzentrischen Halbkugeln 32, 33 mit verschiedenen Durchmessern, wobei die Außenfläche der Innenpfanne 29 in der Nähe der Äquatorebene 34 von der Form der äußeren Halbkugel 33 in die Form eines Zylinders 35 übergeht, der mit der entsprechenden Innenfläche des Pfannenträgers 1 korrespondiert.

In der äußeren Halbkugel 33 ist in der Nähe der Äquatorebene 34 eine umlaufende Nut 31 vorgesehen, die mit der Nut 28 des Pfannenträgers 1 korrespondiert. In die beiden Nuten 28 und 31 ist ein Federdraht oder ein anderes mechanisches Halteelement einsetzbar, das die Innenpfanne 29 am Herausfallen aus dem Pfannenträger 1 hindert.

## Patentansprüche

1. Hüftgelenkspfanne zur zementfreien Implantation in das Acetabulum des Hüftbeins, bestehend aus einem elastischen Pfannenträger, der die Form einer im wesentlichen halbkugelförmigen Schale aufweist und in das entsprechend vorbereitete Acetabulumbett formschlüssig einsetzbar ist, dadurch gekennzeichnet, daß der Pfannenträger (1) in dem im implantierten Zustand nach oben (kranial) gerichteten Bereich (2) seine größte Steifigkeit aufweist, während in Richtung nach unten (kaudal) die Elastizität des Pfannenträgers (1) dadurch zunimmt, daß einerseits die Wandstärke des Pfannenträgers (1) in dem nach oben gerichteten Bereich (2) am größten ist und in Richtung zum unteren Bereich (3) abnimmt und daß im Bereich des Pfannenträgers (1) mit erhöhter Elastizität in verschiedenen Abständen von der Äquatorebene (6) zwei oder mehr parallel oder wenigstens annähernd parallel zu der Äquatorebene (6) verlaufende Einschnitte (11, 12, 13) vorgesehen sind.

2. Hüftgelenkspfanne nach Anspruch 1, dadurch gekennzeichnet, daß die parallel oder wenigstens annähernd parallel zu der Äquatorebene (6) ausgerichteten Einschnitte (11, 12, 13) bis zur Symmetrieachse (7), bis in die Nähe der Symmetrieachse (7) der inneren Halbkugel (4) oder auch geringfügig darüber hinaus in verschiedenen Abständen von der Äquatorebene (6) verlaufen, so daß eine oder mehrere Spangen (SP) gebildet sind, die durch einen radialen Einschnitt (15) in die Spangenteile (11a, 12a, 13a bzw. 11b, 12b, 13b) aufgeteilt sind.

3. Hüftgelenkspfanne nach Anspruch 1 oder 2, dadurch gekennzeichnet, daß die Dicke der Spangenteile (11a, 12a, 13a bzw. 11b, 12b, 13b) beiderseits des radialen Einschnitts (15) jeweils bis zum Ende der Spangenteile (11a, 12a, 13a bzw. 11b, 12b, 13b) zunimmt, vorzugsweise stufenlos zunimmt.

4. Hüftgelenkspfanne nach Anspruch 1, dadurch gekennzeichnet, daß die Neigung (α) der Einschnitte (11, 12, 13) zu der Äquatorebene (6) bis zu maximal ± 10° beträgt.

5. Hüftgelenkspfanne nach einem der Ansprüche 1 bis 4, dadurch gekennzeichnet, daß die Innenfläche und die Außenfläche des Pfannenträgers (1) die Form von Halbkugeln (4, 5) mit sich deckenden Äquatorebenen (6) , jedoch mit unterschiedlichen Durchmessern, und mit gegeneinander parallelverschobenen Symmetrieachsen (7, 8) aufweisen.

6. Hüftgelenkspfanne nach einem der Ansprüche 1 bis 5, dadurch gekennzeichnet, daß die Breite (B) der Spangenteile (11a, 12a, 13a, bzw. 11b, 12b, 13b) in Richtung zur Äquatorebene (6) zunimmt.

7. Hüftgelenkspfanne nach einem der Ansprüche 1 bis 6, dadurch gekennzeichnet, daß die obere Schnittfläche (10) des obersten Einschnitts (11) die innere Halbkugelfläche (4) im Pfannenzentrum (PZ) tangential berührt.

8. Hüftgelenkspfanne nach einem der Ansprüche 1 bis 3, dadurch gekennzeichnet, daß der radial verlaufende Einschnitt (15) sich nur bis zum obersten, parallel zur Äquatorebene (6) verlaufenden Einschnitt (11) erstreckt, so daß das Pfannenzentrum (PZ) auch im Bereich des Pfannenträgers (1) mit erhöhter Elastizität eine einschnittlose, geschlossene Fläche bildet (Fig. 4).

9. Hüftgelenkspfanne nach einem der Ansprüche 1 bis 8, dadurch gekennzeichnet, daß an dem oberhalb der Symmetrieachse (8) der äußeren Halbkugel (5) liegenden Teil des Pfannenträgers (1) ein sich vom Rand aus nach außen erstreckender Kragen (21) vorgesehen ist.

10. Hüftgelenkspfanne nach Anspruch 9, dadurch gekennzeichnet, daß der Kragen (21) im Bereich der radialen Endflächen (25) durch zwei parallele Flächen (26) begrenzt ist, deren Abstand voneinander gleich wie oder nur geringfügig größer als der Außendurchmesser des Pfannenträgers (1) ist.

11. Hüftgelenkspfanne nach einem der Ansprüche 1 bis 10, dadurch gekennzeichnet, daß die Innenfläche des Pfannenträgers (1) in der Nähe der Äquatorebene (6) von der Form der inneren Halbkugel (4) in die Form eines Zylinders (14) übergeht, dessen Symmetrieachse mit der Symmetrieachse (7) der inneren Halbkugel (4) zusammenfällt.

12. Hüftgelenkspfanne nach einem der Ansprüche 1 bis 11, dadurch gekennzeichnet, daß die Innenfläche des Pfannenträgers (1) eine in geringem Abstand von der Äquatorebene (6) verlaufende Nut (28) aufweist.

13. Hüftgelenkspfanne nach einem der Ansprüche 1 bis 12, dadurch gekennzeichnet, daß der Pfannenträger (1) einen zur Symmetrieachse (7) der inneren Halbkugel (4) symmetrischen Durchbruch (9) geringen Durchmessers bis zu etwa 5 mm zur vorläufigen Fixierung im Acetabulumbett aufweist.

14. Hüftgelenkspfanne nach einem der Ansprüche 1 bis 13, dadurch gekennzeichnet, daß der Pfannenträger (1) aus Metall, vorzugsweise aus Titan, Titanlegierungen oder aus anderen körperverträglichen Metallen besteht.

15. Hüftgelenkspfanne nach Anspruch 14, dadurch gekennzeichnet, daß die Oberfläche des Pfannenträgers (1) auf der Außenseite, insbesondere in dem nach oben gerichteten Bereich (2), mit Oxiden, Nitriden oder Oxinitriden, sogenannter "Ventilmetalle", insbesondere mit Mischmetalloxidschichten aus Titan-Zirkon, Titan-Niob, Titan-Tantal, Titan-Haffnium, Titan-Wolfram, Titan-Tantal-Zirkon, Titan-Tantal-Niob, Titan-Tantal-Haffnium, Zirkon-Niob, Niob-Haffnium oder Tantal-Zirkon, insbesondere nach vorherigem Aufrauhen durch Sandstrahlen oder andere mechanische Methoden, wie dem PVD (Physical Vapour Deposition) Verfahren, beschichtet ist.

16. Hüftgelenkspfanne nach einem der Ansprüche 1 bis 15, dadurch gekennzeichnet, daß der Pfannenträger (1) durch zerspanende Arbeitsgänge aus Vollmaterial hergestellt ist.

17. Hüftgelenkspfanne nach einem der Ansprüche 1 bis 16, dadurch gekennzeichnet, daß in den Pfannenträger (1) eine Innenpfanne (29) aus Kunststoff, vorzugsweise aus Polyäthylen, formschlüssig einsetzbar ist, die durch einen auf dem Rand des Pfannenträgers (1) aufliegenden Kragen (30) am Verschwenken um die in der Äquatorebene (34) liegenden Achsen gehindert ist.

## Claims

1. A hipjoint socket for cementfree implantation in the acetabulum of
the hipbone, consisting of an elastic socket-carrier which exhibits the shape of an essentially hemispherical cup and may be inserted with a closed fit in the correspondingly prepared bed in the acetabulum, characterized in that the socket-carrier (1) in the region (2) which in the implanted state is directed upwards (cranially) exhibits its greatest stiffness, whilst in the downwards (caudal) direction the elasticity of the socket-carrier (1) increases, that on one side the wall thickness of the socket-carrier (1) is greatest in the upwards directed region (2) and decrases in the direction of the lower region (3) and that two or more notches (11, 12, 13) are provided in the region of the socket-carrier (1) of increased elasticity, running in parallel or at least approximately in parallel with the equatorial plane (6) and at different distances from the equatorial plane (6).

2. A hipjoint socket as in Claim 1, characterized
in that the notches (11, 12, 13) which are aligned to parallel or at least approximately to parallel with the equatorial plane (6) run at different distances from the equatorial plane (6) up to the axis (7) of symmetry, close to the axis of symmetry of be inner hemisphere (4) or even slightly beyond it so that one or more clasps (SP) are formed which are subdivided by a radial notch (15) into the clasp parts (11a, 12a, 13a resp. 11b, 12b, 13b).

3. A hipjoint socket as in Claim 1 or 2, characterized
in that the thickness of each of the clasp parts (11a, 12a, 13a resp. 11b, 12b, 13b) increases on both sides of the radial notch (15) up to the ends of the clasp parts (11a, 12a, 13a resp. 11b, 12b, 13b), preferably increasing continuously.

4. A hipjoint socket as in Claim 1, characterized
in that the inclination (α) of the notches (11, 12, 13) with respect to the equatorial plane (6) amounts to a maximum of ± 10°.

5. A hipjoint socket as in one of the Claims 1 to 4, characterized
in that the inner and outer faces of the socket-carrier (1) exhibit the shape of hemispheres (4, 5) with coincident equatorial planes (6) but of different diameters and with axes (7, 8) of symmetry displaced in parallel with one another.

6. A hipjoint socket as in one of the Claims 1 to 5, characterized
in that the width (B) of the clasp parts (11a, 12a, 13a, resp. 11b, 12b, 13b) increases in the direction of the equatorial plane (6).

7. A hipjoint socket as in one of the Claims 1 to 6, characterized
in that the upper cut face (10) of the uppermost notch (11) touches the inner hemispherical face (4) tangentially at the centre (PZ) of the socket.

8. A hipjoint socket as in one of the Claims 1 to 3, characterized
in that the notch (15) running radially extends only up to the uppermost notch (11) running in parallel with the equatorial plane (6), so that even in the region of incrased elasticity of the socket-carrier (1) the centre (PZ) of the socket forms a closed araea (Figure 4).

9. A hipjoint socket as in one of the Claims 1 to 8, characterized
in that on the part of the socket-carrier (1) lying above the axis of symmetry (8) of the outer hemisphere (5) a collar (21) is provided which extends outwards from the edge.

10. A hipjoint socket as in Claim 9, characterized
in that the collar (21) is bounded in the region of its radial endfaces (25) by two parallel faces (26), the distance apart of which is equal to or slightly greater than the outer diameter of the socket-carrier (1).

11. A hipjoint socket as in one of the Claims 1 to 10, characterized
in that in the neighbourhood of the equatorial plane (6) the inner face of the socket-carrier (1) continues from the shape of the inner hemisphere (4) into the shape of a cylinder (14) the axis of symmetry of which coincides with the axis of symmetry (7) of the inner hemisphere (4).

12. A hipjoint socket as in one of the Claims 1 to 11, characterized
in that the inner face of the socket-carrier (1) exhibits a groove (28) running at a short distance from the equatorial plane (6).

13. A hipjoint socket as in one of the Claims 1 to 12, characterized
in that the socket-carrier (1) exhibits an opening (9) of small diameter up to about 5 mm, symmetrical about the axis of symmetry (7) of the inner hemisphere (4), for preliminary fixing in the bed in the acetabulum.

14. A hipjoint socket as in one of the Claims 1 to 13, characterized
in that the socket-carrier (1) consists of metal, preferably titanium or titanium alloys or other metals compatible with the human body.

15. A hipjoint socket as in Claim 14, characterized
in that the surface of the socket-carrier (1) is coated on the outside, in particular in the upwards directed region (2), with oxides, nitrides or oxinitrides of socalled "valve metals", in particular with mixed metal oxide layers of titanium-zirconium, titanium-niobium, titanium-tantalum, titanium-hafnium, titanium-tungsten, titanium-tantalum-zirconium, titanium-tantalum-niobium, titanium-tantalum-hafnium, zirconium-niobium, niobium-hafnium or tantalum-zirconium, in particular after previous roughening by sandblasting or by other mechanical methods such as the PVD (physical vapour deposition) method.

16. A hipjoint socket as in one of the Claims 1 to 15, characterized
in that the socket-carrier (1) is produced from solid material by cutting operations.

17. A hipjoint socket as in one of the Claims 1 to 16, characterized
in that there may be inserted in the socket-carrier (1) an inner socket (29) of plastics, preferably polyethylene, which is impeded by a collar (30) resting against the edge of the socket-carrier (1) from swivelling about the axes lying in the equatorial plane (34).

## Revendications

1. Cuvette d'articulation de la hanche pour l'implantation sans ciment dans la cavité acétabulaire, se composant d'un porte cuvette, qui a la forme d'une coquille essentiellement en forme de demi-sphère et peut être inséré dans la cavité acétabulaire essentiellement préparée de façon adéquate pour une liaison par la forme, caractérisée en ce que la porte cuvette (1) a sa plus grande rigidité dans la zone (2) dirigée à l'état implanté vers le haut (craniale), tandis que l'élasticité du porte cuvette (1) augmenté en direction du bas (caudale), du fait que d'une part l'épaisseur de la paroi du porte cuvette (1) est la plus importante dans la zone (2) dirigée vers le haut et diminue dans la direction de la zone (3) inférieure et du fait que dans la zone du porte cuvette (1) à plus forte élasticité sont prévues à différentes distances du plan axial (6) deux découpures ou plus (11, 12, 13) se développant parallèlement ou au moins approximativement parallèlement au plan axial (6).

2. Cuvette d'articulation de la hanche selon la revendication 1, caractérisée en ce que les découpures (11, 12, 13) dirigées parallèlement ou approximativement parallèlement au plan axial (6) s'étendent à des distances différentes du plan axial (6) jusqu'à l'axe de symétrie (7), jusqu'au voisinage de l'axe de symétrie (7) de la demi-sphère intérieure (4) ou même légèrement au delà, de sorte que sont constituées une ou plusieurs barrettes (SP), qui sont partagées par une découpure radiale (15) dans les parties de barrettes (11a, 12a, 13a ou 11b, 12b, 13b).

3. Cuvette d'articulation de la hanche selon la revendication 1 ou 2 caractérisée en ce que l'épaisseur des parties de barrettes (11a, 12a, 13a ou 11b, 12b, 13b) des deux côtés de la découpure (15) augmente respectivement jusqu'à l'extrémité des parties de barrette (11a, 12a, 13a ou 11b, 12b, 13b), de préférence elle augmente de façon progressive.

4. Cuvette d'articulation de la hanche selon la revendication 1, caractérisée en ce que l'inclinaison (α) des découpures (11, 12, 13) par rapport au plan axial (6) peut atteindre au maximum ± 10°.

5. Cuvette d'articulation de la hanche selon une des revendications 1 à 4, caractérisée en ce que la face intérieure et la face extérieure du porte cuvette (1) ont la forme hémisphérique (4, 5) avec des plans axiaux (6) se recouvrant, toutefois avec des diamètres différents et avec des axes de symétrie (7, 8) décalés parallèlement l'un par rapport à l'autre.

6. Cuvette d'articulation de la hanche selon une des revendications 1 à 5, caractérisée en ce que la largeur (13) des barrettes (11a, 12a, 13a ou 11b, 12b, 13b) augmente en direction du plan axial (6).

7. Cuvette d'articulation de la hanche selon une des revendications 1 à 6, caractérisée en ce que la surface de coupe supérieure (10) de la découpure la plus haute (11) a un contact tangentiel avec la face de demi-sphère intérieure (4) au centre de la cuvette (PZ).

8. Cuvette d'articulation de la hanche selon une des revendications 1 à 3, caractérisée en ce que la découpure (15) se développant radialement s'étend seulement jusqu'à la découpure (11) supérieure en se développant parallèlement au plan axial (6), de sorte que le centre de la cuvette (PZ) forme aussi dans la zone du porte cuvette (1) avec une élasticité accrue une surface sans découpure, fermée.

9. Cuvette d'articulation de la hanche selon une des revendications 1 à 8, caractérisée en ce que sur la partie du porte cuvette (1) situé au-dessus de l'axe de symétrie (8) de la demi-sphère extérieure (5) est prévu un collet (21) s'étendant du bord vers l'extérieur.

10. Cuvette d'articulation de la hanche selon la revendication 9, caractérisé en ce que le collet (21) est délimité au niveau des faces terminales radiales (25) par deux faces parallèles (26), dont la distance de l'une à l'autre est égale ou légèrement plus grande que le diamètre extérieur du porte cuvette (1).

11. Cuvette d'articulation de la hanche selon une des revendications 1 à 10, caractérisée en ce que la surface intérieure du porte cuvette (1) passe dans la zone du plan axial (6), de la forme de la demi-sphère intérieure (4) à la forme d'un cylindre (14) dont l'axe de synthèse coïncide avec l'axe de symétrie (7) de la demi-sphère intérieure (4).

12. Cuvette d'articulation de la hanche selon une des revendications 1 à 11, caractérisée en ce que la surface intérieure du porte cuvette (1) comporte une rainure (28) se développant à une faible distance du plan axial (6).

13. Cuvette d'articulation de la hanche selon une des revendications 1 à 12, caractérisée en ce que le porte cavité (1) a un percement (9) symétrique par rapport à l'axe de symétrie (7) de la demi-sphère intérieure (4), avec un faible diamètre jusqu'à environ 5 mm pour la fixation provisoire dans la cavité acétabulaire.

14. Cuvette d'articulation de la hanche selon une des revendications 1 à 13 caractérisée en ce que le porte cuvette (1) est en métal de préférence en titane, alliages de titane ou en d'autres métaux supportables pour le corps.

15. Cuvette d'articulation de la hanche selon la revendication 14, caractérisée en ce que la surface du porte cuvette (1) est recouverte sur la face extérieure, en particulier dans la zone (2) dirigée vers le haut, d'oxydes, nitrures ou oxynitrures, de "métaux dits de soupape", en particulier de couches d'oxydes métalliques mixtes de titane-zirconium, titane-niobium, titane-tantale, titane-haffnium, titane-tungstène, titane-tantale-zirconium, titane-tantale-niobium, titane-tantale-haffnium, zirconium-niobium, niobium-haffnium ou tantale-zirconium, en particulier après l'avoir rendue rugueuse par des jets de sable ou autres méthodes mécaniques, comme le procédé PVD (Physical Vapour Deposition).

16. Cuvette d'articulation de la hanche selon une des revendications 1 à 15, caractérisée en ce que le porte cuvette (1) est fabriqué par des opérations d'usinage à partir d'un matériau plein.

17. Cuvette d'articulation de la hanche selon une des revendications 1 à 15, caractérisée en ce qu'on peut utiliser en liaison par la forme dans le porte cuvette (1) une cuvette intérieure (29) en matière synthétique, de préférence en polyéthylène, cuvette qui se trouve empêchée de pivoter autour des axes situés dans le plan axial (34) par un collet (30) en saillie sur le bord du porte cuvette (1).
